# EUROPÄISCHE PATENTSCHRIFT

(11) **EP 0 667 960 B1**
(45) Veröffentlichungstag und Bekanntmachung des Hinweises auf die Patenterteilung: **02.04.1997**
(21) Anmeldenummer: 94900090.5
(22) Anmeldetag: 03.11.1993
(51) Int. Cl.: G01N 33/543, C12Q 1/68, C12N 15/11

(54) **TRÄGERMATERIAL ZUR SIMULTANEN BINDUNG VON NUKLEINSÄUREN UND DEREN EXPRESSIONSPRODUKTEN**
SUPPPORT MATERIAL FOR SIMULTANEOUS BINDING OF NUCLEIC ACIDS AND EXPRESSIONPRODUCTS THEREOF
SUPPORT POUR FIXER SIMULTANEMENT DES ACIDES NUCLEIQUES ET DE LEURS PRODUITS D'EXPRESSION

(30) Priorität: 05.11.1992 DE 4237381
(43) Veröffentlichungstag der Anmeldung: 23.08.1995
(73) Patentinhaber: EVOTEC BIOSYSTEMS GMBH, D-22529 Hamburg (DE)
(72) Erfinder: HENCO, Karsten, D-40699 Erkrath (DE); EIGEN, Manfred, D-37075 Göttingen (DE)
(74) Vertreter: Meyers, Hans-Wilhelm, Dr.Dipl.-Chem.
(86) Internationale Anmeldenummer: EP9303075
(87) Internationale Veröffentlichungsnummer: WO9410572

(56) Entgegenhaltungen:
- WO-A-92/18645
- DE-A- 3 717 209
- DE-A- 3 717 210
- DATABASE WPI Section Ch, Week 9023, Derwent Publications Ltd., London, GB; Class A96, AN 90-171939 HUNGER, H.D. ET AL. 'Production of hydrophobic-ionic polymer derivatives - useful for dewtection and separation in molecular biology, etc.' & DD,A,274 676 (AKADEMIE DER WISSENSCHAFTEN DER DDR) 27. Dezember 1989
- BERICHTE DER BUNSEN-GESELLSCHAFT FüR PHYSIKALISCHE CHEMIE Bd. 89, Nr. 6 , Juni 1985 , WEINHEIM DE Seiten 658 - 667 MANFRED EIGEN 'Macromolecular Evolution: Dynamical Ordering in Sequence Space'

## Beschreibung

Die Erfindung betrifft ein Trägermaterial sowie ein Verfahren zur evolutiven Optimierung von Biopolymeren.

Die evolutive Biotechnologie ist eine neuartige Technik, mit der quasi im Reagenzglas die Evolution nachgestellt wird, um allein durch Replikationsmechanismen mutierte und optimierte Nukleinsäurestrukturen zu erhalten. Die Vermehrung der Nukleinsäure erfolgt mit entsprechenden Enzymsystemen, wie Polymerasen, deren natürliche Ablesefehlerhäufigkeit dazu benutzt wird, eine Quasi- Spezies-Verteilung zu schaffen ausgehend von einem bestimmten Nukleinsäuremolekül oder Ensemble. Genaueres über diese Technik wird in der DE-PS 41 12 440 C 2 beschrieben.

Gemeinsam ist den Techniken der evolutiven Biotechnologie jedoch, daß eine große Anzahl von Proben parallel bearbeitet werden muß, um den Zeitrahmen der evolutiven Optimierung technisch einsetzbar zu gestalten. So wird angestrebt, beispielsweise einen bestimmten Phänotyp, beispielsweise ein Enzym, über Mutageneseverfahren des kodierenden Gens systematisch zu optimieren. Ein solcher Prozeß, der nach dem Verfahren des sogenannten irrationalen oder evolutiven Designs arbeitet, verlangt also ein Durchmustern der erhaltenen Phänotypen auf der Basis bestimmter erwünschter Funktionen. Wenn ein erwünschter Phänotyp gefunden wird, gilt es, unmittelbar einen Zugriff auf das kodierende Gen, also die entsprechende Nukleinsäure, zu haben. Indirekte Wege, um zur kodierenden Sequenz zu gelangen, beispielsweise über eine Proteinsequenz-analyse und Transferierung in die entsprechende Gensequenz, sind technisch zwar möglich, jedoch aufgrund der recht aufwendigen Technik in der Praxis kaum durchführbar.

Es besteht zur technisch vernünftigen Durchführung der evolutiven Biotechnologie auch die Notwendigkeit, parallel für eine Vielzahl einzelner, transformierter Zellen eine lokal fixierte spezifische Reinigung von Phänotyp und Genotyp in einem Trennprozeß durchzuführen, d.h. etwa 10⁶ -10⁸ Experimente parallel durchzuführen.

Der Erfindung liegt mithin das technische Problem zugrunde, ein System zur Verfügung zu stellen, das eine gemeinsame Reinigung eines Phänotypen und eines Genotypen, nämlich eines Proteins und seines entsprechenden kodierenden Gens, erlaubt und damit ein Verfahren zur Verfügung zu stellen, das eine evolutive Optimierung von Biopolymeren erlaubt. Dieses Trägersystem soll in Kombination mit einem Wachstumsmedium gleichzeitig ein lokalisiertes Wachstum einer Zellkolonie erlauben oder ein entsprechendes enzymatisches Genamplifikationssystem einsetzbar gestalten, dessen Genotyp mit dem Phänotyp lokal gekoppelt bleibt und in einem lokalisierten Element verbleibt, ohne sich mit anderen Geno- oder Phänotypen zu vermischen.

Gelöst wird dieses Problem durch ein Trägermaterial, das simultan mindestens zwei Bindeeigenschaften aufweist zur jeweiligen spezifischen Bindung von Nukleinsäuren und deren Expressionsprodukten, wobei bestimmte Areale der Oberfläche des Trägermaterials jeweils die Nukleinsäuren binden können, während die Expressionsprodukte von lokal getrennten Arealen der Oberfläche des Trägermaterials gebunden werden und ein Areal Affinitätseigenschaften und ein anderes Areal Ionenaustauscher- oder Affinitätseigenschaften aufweist, oder ein und dasselbe Areal auf dem Trägermaterial die Nukleinsäuren und Expressionsprodukte bindet und ein Bereich des Areals Affinitätseigenschaften und ein anderer Bereich desselben Areals Ionenaustauscher- oder Affinitätseigenschaften aufweist. Dadurch wird vermieden, daß sich verschiedene Gene oder Genotypen ohne weiteres miteinander vermischen und andererseits Enzymsysteme, die als Phänotypen aus den Genotypen (Informationsträgern) hervorgegangen sind, mit ihren jeweiligen Genen assoziiert bleiben und somit grundsätzlich isolierbar sind. Das Trägersystem zeichnet sich dadurch aus, daß das erfindungsgemäße Trägermaterial an bestimmte Areale seiner Oberfläche jeweils die Nukleinsäuren binden kann, während deren Expressionsprodukte von anderen Arealen auf der Oberfläche dieses Trägermaterials gebunden werden.

Das erfindungsgemäße Trägermaterial besteht aus Arealen, die Geno- und/oder Phänotyp zu binden vermögen und einen Abstand von < 1.000 µm, vorzugsweise < als 100 µm voneinander haben, wobei der Abstand 10 nm nicht unterschreiten sollte. Aufgrund der unterschiedlichen chemischen Natur des Geno- und Phänotyps weisen die Areale vorzugsweise gleichzeitig zwei verschiedene Bindeeigenschaften auf, die jeweils spezifisch für die genannten Stoffklassen Nukleinsäuren des Genotyps und deren Expressionsprodukte als Phänotyp sind.

Die Areale weisen erfindungsgemäß Ionenaustauscher-, Affinitätseigenschaften wie hydrophobe Wechselwirkungseigenschaften oder Komplexbildungseigenschaften oder Kombinationen davon auf. So ist es beispielsweise möglich, daß bei Verwendung von Arealen mit Affinitätseigenschaften der Genotyp von bestimmten Affinitätsliganden in den entsprechenden Arealen erkannt wird, wohingegen der Phänotyp mit einem anderen Affinitätsliganden, der ebenfalls in den entsprechenden Arealen vorliegt, in Wechselwirkung treten kann. Die Moleküle oder Molekülklassen, die jeweils Phänotyp oder Genotyp repräsentieren, können also direkt oder über bestimmte natürliche oder künstlich eingeführte Substituenten an die Trägeroberfläche gebunden werden. Solche Substituenten können Haptene sein oder Biotin oder Avidin oder andere entsprechende Seitenketten, wie bestimmte Oligopeptide. Es kann aber auch vorteilhaft sein, ein Trägermaterial zu verwenden, das in einem Bereich der Areale Affinitätseigenschaften und in anderen Bereichen derselben Areale Ionenaustauschereigenschaften aufweist.

Das erfindungsgemäße Trägermaterial sollte bei mit Affinitätsliganden modifizierter Oberfläche reversible Wechselwirkungen mit den gebundenen Geno- oder Phänotypen erlauben, wodurch die Elution mindestens eines der gebundenen Molekültypen (Geno- oder Phänotyp) ermöglicht wird.

Das Trägermaterial, das die Areale mit den Bindungseigenschaften für Geno- und Phänotyp besitzt, ist vorzugsweise auf oder in einer Matrix wie einer Membran oder einem Membranverbund, einer Fläche, einem Wafer, einer Kapillare, Fasern oder Faserverbund wie gewebten Teilen oder Vliesen oder Kombinationen dieser Gegenstände angeordnet.

Es kann auch vorteilhaft sein, ein Trägermaterial mit Arealen zur Verfügung zu stellen, welches eine Affinität aufweist zu mindestens einem Protein oder Peptid, aber gleichzeitig auch Nukleinsäuren binden kann. Ein solches Material kann praktisch als Hybrid eines Ionenaustauschers und affinitätschromatographischen Materials aufgefaßt werden. Diese gruppenspezifische Bindeeigenschaft für Nukleinsäuren und Proteine ermöglicht also die gleichzeitige Bindung von Nukleinsäuren und Proteinen. Über die spezifische Proteinbindung, die innerhalb eines bestimmten Volumenelements nur in definierter Anzahl erfolgen kann, wird eine Standardisierung ermöglicht.

Vorzugsweise werden als Ionenaustauscher für die Areale des erfindungsgemäßen Trägermaterials großporige oder nichtporige Materialen verwendet, die noch bei einer Ionenstärke vergleichbar einer 1 M Natriumchloridkonzentration Polyanionen, wie DNA, zu binden vermögen. Insbesondere haben sich großporige Silicagele, wie sie in der DE 32 11 309 beschrieben werden und unter der Bezeichnung QIAGEN im Handel erhältlich sind, bewährt. Affinitätsliganden können beispielsweise Biotinderivate und/oder Streptavidinderivate und/oder Avidinderivate und/oder Nukleinsäuren und/oder Antikörperfragmente und/oder Metallchelate und/oder Peptidliganden sein. Wenn Anionenaustauschermaterialien und Affinitätsmaterialien auf unterschiedlichen partikulären Trägern gekoppelt sind, können beide Partikelsorten innig vermischt werden, so daß beide Bindeeigenschaften in den Arealen des erfindungsgemäßen Trägers zu finden sind. Sind die Areale auf faserigen Trägern angeordnet, so läßt sich der gleiche Effekt auch durch Verweben der Fasern oder gemeinsames Pressen der Fasern erzielen oder auch durch einen engen Zusammenschluß dünner Membranen, von denen zumindest eine Membran für mindestens eine Substanzklasse passierbar sein muß. Es kann jedoch auch ein netzartiger Träger Verwendung finden, der auf beiden Seiten jeweils Bindeeigenschaften für eine Molekülsorte, Geno- oder Phänotyp, aufweist.

Die Verwendung der erfindungsgemäßen Trägermaterialien dient der engen räumlichen Bindung zweier unterschiedlicher Moleküle, wie beispielsweise Proteine und/oder Nukleinsäuren, die auch für die entsprechenden Proteine kodieren können. Besonders vorteilhaft läßt sich der erfindungsgemäße Träger zur evolutiven Optimierung von Biopolymeren einsetzen, insbesondere zur parallelen Analyse unterschiedlicher Genprodukte und ihrer zugehörigen Gene.

Es wird mithin ein Verfahren zur evolutiven Optimierung von Biopolymeren zur Verfügung gestellt, wobei gleichzeitig Genotyp und Phänotyp an das erfindungsgemäße Trägermaterial bindbar sind und selektiv oder gleichzeitig isoliert werden können.

Das erfindungsgemäße Verfahren läuft vorzugsweise in einem Reaktionsraum ab, in dem der den Genotyp repräsentierende Strukturtyp, beispielsweise eine Nukleinsäure, einer Mutagenese unterworfen wird, dieser Strukturtyp mindestens einem Replikationsschritt unterworfen wird, in einem Expressions-system exprimiert wird und das entsprechende Expressionsprodukt (Phänotyp), beispielsweise ein Protein, zusammen mit dem Genotyp, beispielsweise einer für das Protein kodierenden Nukleinsäure, an dem Trägermaterial gebunden und auf spezifische optimierte Eigenschaften untersucht wird.

Proteine und/oder Nukleinsäuren lassen sich zu Testzwecken und zur weiteren Bearbeitung nutzen. Als besonders vorteilhaft erweist sich die Kombination von Metallchelatchromatographie auf der Basis von Nickel-NTA-gekoppelten Festphasenträgern, die in der Lage sind, Proteine, mit 6 Histidinen in Folge, aus wäßriger Lösung zu extrahieren und komplex zu binden, sowie der Anionenaustauscherchromatographie zur Extraktion und Reinigung von Nukleinsäuren. Diese Kombination kann insbesondere auf einer Membran oder einem eindimensionalen Gewebe angeordnet werden, womit sich, in Gegenwart eines entsprechenden Kulturmediums, Zellkolonien lokalisieren und voneinander getrennt kultivieren lassen.

Als Replikationssystem für Gene können jedoch anstelle von Zellen auch zellfreie Systeme, wie enzymatische Amplifikationssysteme, eingesetzt werden. Dabei dienen die Areale des erfindungsgemäßen Trägers praktisch als Kompartimentierungen, in denen Reaktionen zwischen Reaktionspartnern ablaufen können, ohne jeweils mit den anderen Arealen in Wechselwirkung zu treten. Dies wird durch die Beteiligung der Oberflächen gewährleistet, wobei Diffusionsprozesse der Makromoleküle unterbunden werden. Es lassen sich also enzymatische Amplifikationen durchführen, ohne daß sich die Amplifikationsprodukte unterschiedlicher Mastersequenzen miteinander vermischen. Dadurch wird die Etablierung einer Quasi-Spezies-Verteilung, begrenzt auf den räumlichen Bereich der entsprechenden Areale, ermöglicht.

Als besonders geeignete Ausführungsform hat sich die folgende Membran bewährt. Auf dieser Membran sind bestimmte Areale vorhanden, die vorzugsweise aus Anionenaustauschermaterial gemäß der DE-A-32 11 309 C 2 und Materialien, die mit Nitrilotriessigsäure und/oder den entsprechenden Nickelchelaten dieser Stoffgruppe modifiziert sind. Die Membran wurde nach dem sogenannten enmesh-Verfahren (der Firma 3M, St. Paul, USA) verarbeitet. Das erfindungsgemäße Trägermaterial kann in einem Verfahren eingesetzt werden, was im einzelnen folgendes erlaubt:
- vergleichende Screenings von ungefähr 10⁶ - 10⁸ Klonen in einem Experiment,
- Phänotyp/Genotyp-Kopplung, d.h. der direkte Zugriff von einem als optimal charakterisierten Protein auf das dementsprechende Gen,
- den quantifizierenden Vergleich enzymatischer Aktivitäten zellständiger oder sezernierter Proteine,
- Einsatz prokaryotischer oder eukaryotischer Zellen,
- automatische Handhabung und Überprüfung auf bestimmte Eigenschaften,
- Standardisierung rekombinanter Proteine,
- Rückgewinnung der spezifisch gebundenen Proteine und Nukleinsäuren sowie
- strategisch geführte evolutionäre Verbesserung von Proteinstrukturen.

Der vorteilhafte Einsatz des erfindungsgemäßen Trägermaterials wird anhand des folgenden Beispiels näher erläutert:

Eine Membran, die mit Kulturmedium gesättigt ist, dient zur Aufnahme rekombinanter Zellen. Nach Transformation kompetenter Escherischia-coli-Zellen und Bestimmung der Konzentration der kompetenten Zellen im Medium, wird die Zellsuspension mit einem Piezo-Pipettierautomaten so auf die Membran aufgetragen, daß statistisch eine Zelle pro Tröpfchen und Flächenelement abgelegt wird. Dieses Flächenelement wird je nach Menge der zu positionierenden Zellen in einem Raster mit einem Abstand von 0,1 - 1 mm gewählt. So lassen sich 10⁶ - 10⁸ Zellen pro m² positionieren. Als kompetente Zellen kommen auch Bacillus subtilis oder Baculovirus infizierte Insektenzellen infrage.

Die transformierten kompetenten Zellen werden für eine Zeitdauer von etwa 20 Replikationszyklen kultiviert, so daß in etwa 10⁶ Zellen aus einer rekombinanten Zelle hervorgehen. Danach erfolgt die Induktion des rekombinanten Proteins entweder durch chemische oder thermische Induktion unter Weiterführung der Kultivierung für einen Zeitraum von ca. 1 - 12 Stunden. Die transformierten Nukleinsäuren kodieren in dem entsprechenden Protein für eine Aminosäuresequenz, die 6 Histidinreste aufweist. Sobald diese Proteine sezerniert werden, werden die so markierten Proteine durch die Areale mit Nitrilotetraessigsäure modifizierten Oberflächen gebunden. Die entsprechende Nukleinsäure wird ebenfalls nach Freisetzung durch Lösen der Zellen, allerdings auf dem Ionenaustauschermaterial, welches sich in dem Areal des erfindungsgemäßen Trägers befindet, gebunden.

Die Membran wird zur Entfernung von Zelltrümmern und anderen Bestandteilen gewaschen und vermessen.

Das Ionenaustauschermaterial QIAGEN weist den besonderen Vorteil auf, daß es in der Lage ist Nukleinsäuren zu binden, ohne daß auch unter ungünstigen äußeren Bedingungen die gebundene Nukleinsäure abgebaut wird. Die gebundene Nukleinsäure, beispielsweise DNA, wird erst durch Elution mit hohen Salzkonzentrationen freigesetzt.

Nach der Vermessung der Membran werden die Nukleinsäuren der Mutanten mit der höchsten Aktivität aus den entsprechenden Arealen eluiert und können für einen weiteren Zyklus in einem evolutiven Biotechnologieverfahren eingesetzt werden.

Durch Kombination mit automatischen Sequenzierverfahren erlaubt das erfindungsgemäße Verfahren eine gute Abschätzung der Fitness-Landschaft, die eine bestimmte Mutante umgibt.

## Patentansprüche

1. Trägermaterial, das simultan mindestens zwei Bindeeigenschaften aufweist zur jeweiligen spezifischen Bindung von Nukleinsäuren und deren Expressionsprodukten, wobei bestimmte Areale der Oberfläche des Trägermaterials jeweils die Nukleinsäuren binden können, während die Expressionsprodukte von lokal getrennten Arealen der Oberfläche des Trägermaterials gebunden werden und ein Areal Affinitätseigenschaften und ein anderes Areal Ionenaustauscher- oder Affinitätseigenschaften aufweist, oder ein und dasselbe Areal auf dem Trägermaterial die Nukleinsäuren und Expressionsprodukte bindet und ein Bereich des Areals Affinitätseigenschaften und ein anderer Bereich desselben Areals Ionenaustauscher- oder Affinitätseigenschaften aufweist.

2. Trägermaterial nach Anspruch 1, dadurch gekennzeichnet, daß die jeweiligen Areale zur Bindung der Nukleinsäuren und deren Expressionsprodukten einen Abstand von 10 bis 1000 µm, vorzugsweise 10 nm bis 100 µm, voneinander haben.

3. Trägermaterial nach einem der Ansprüche 1 und/oder 2, dadurch gekennzeichnet, daß das Areal mit Affinitätseigenschaften reversible Wechselwirkungen mit den gebundenen Nukleinsäuren oder deren Expressionsprodukten erlaubt, wodurch eine Elution mindestens eines gebundenen Molekültyps ermöglicht wird.

4. Trägermaterial nach mindestens einem der Ansprüche 1 bis 3, dadurch gekennzeichnet, daß das Trägermaterial flächig auf einer Membran oder einem Membranverbund oder in einer Membran eingebettet, in einer Kapillare oder auf einer Faser oder in einem Faserverbund, wie Vliesen oder Kombinationen davon angeordnet ist.

5. Trägermaterial nach mindestens einem der Ansprüche 1 bis 4, dadurch gekennzeichnet, daß das Material mit Affinitätseigenschaften und das Anionenaustauschermaterial in einer Matrix, wie Vliesen, angeordnet ist.

6. Trägermaterial nach mindestens einem der Ansprüche 1 bis 5, dadurch gekennzeichnet, daß das Areal mit Affinitätseigenschaften zur spezifischen Bindung mindestens eines Proteins oder Peptids fähig ist und gleichzeitig Nukleinsäuren binden kann.

7. Trägermaterial nach mindestens einem der Ansprüche 1 bis 6, dadurch gekennzeichnet, daß die Areale mit Anionenaustauschereigenschaften durch oberflächlich modifizierte groß- oder geschlossenenporige Partikel vermittelt werden.

8. Trägermaterial nach Anspruch 7, dadurch gekennzeichnet, daß das Areal mit Anionenaustauschereigenschaften noch bei einer Ionenstärke entsprechend 1 M NaCl DNA-Moleküle zu binden vermag.

9. Trägermaterial nach mindestens einem der Ansprüche 1 bis 8, dadurch gekennzeichnet, daß das Areal mit Affinitätseigenschaften durch Biotinderivate, Streptavidinderivate, Avidinderivate, Nukleinsäuren, Antikörperfragmente, Metallchelate, Protein und/oder Peptidliganden gebildet wird.

10. Trägermaterial nach mindestens einem der Ansprüche 1 bis 9, dadurch gekennzeichnet, daß das Areal mit Affinitätseigenschaften durch Metallchelate auf Basis von Nickel/ Nitrilotriessigsäure Chelate gebildet werden und die Areale mit Anionenaustauschereigenschaften durch Materialien gebildet werden, die zur Bindung von Nukleinsäuren geeignet sind.

11. Verfahren zur evolutiven Optimierung von Biopolymeren, wobei gleichzeitig Nukleinsäuren und deren Expressionsprodukte an ein Trägermaterial gebunden werden und selektiv oder gleichzeitig isoliert werden unter Verwendung eines Trägermaterials nach mindestens einem der Ansprüche 1 bis 10.

12. Verfahren nach Anspruch 11, wobei in einem Reaktionsraum der den Genotyp repräsentierende Strukturtyp einer Mutagenese unterworfen wird,
der Strukturtyp mindestens einem Replikationsschritt unterworfen, exprimiert,
das Expressionsprodukt zusammen mit den Nukleinsäuren an das Trägermaterial gebunden und untersucht wird.

## Claims

1. A substrate material having at least two simultaneous binding properties for the respective specific binding of nucleic acids and their expression products wherein particular surface areas of the substrate material are capable of binding said nucleic acids whereas said expression products are bound by locally separated surface areas of the substrate material, and one area has affinity properties and another area has ion-exchanging or affinity properties, or one and the same area on the substrate material binds both nucleic acids and expression products and one portion of the area has affinity properties and another portion of the same area has ion-exchanging or affinity properties.

2. The substrate material according to claim 1, characterized in that the respective areas for binding said nucleic acids and their expression products are distanced from one another by 10 to 1000 µm, preferably 10 nm to 100 µm.

3. The substrate material according to any of claims 1 and/or 2, characterized in that the area having affinity properties allows for reversible interactions with the bound nucleic acids or their expression products thereby enabling elution of at least one of the molecular types bound.

4. The substrate material according to at least one of claims 1 to 3, characterized in that said substrate material is located two-dimensionally on a membrane or assembly of membranes or embedded within a membrane, or is located in a capillary or on a fiber or fibrous composite structure, such as non-woven fabrics, or combinations thereof.

5. The substrate material according to at least one of claims 1 to 4, characterized in that the material having affinity properties and the anion-exchanging material are located in a matrix, such as non-woven fabrics.

6. The substrate material according to at least one of claims 1 to 5, characterized in that the area having affinity properties is capable of specifically binding at least one protein or peptide and of binding nucleic acids simultaneously.

7. The substrate material according to at least one of claims 1 to 6, characterized in that the areas having anion-exchanging properties are provided by surface-modified large-pore or closed-pore particles.

8. The substrate material according to claim 7, characterized in that the area having anion-exchanging properties is still capable of binding DNA molecules at an ionic strength corresponding to that of 1 M NaCl.

9. The substrate material according to at least one of claims 1 to 8, characterized in that the area having affinity properties is constituted by biotin derivatives, streptavidin derivatives, avidin derivatives, nucleic acids, antibody fragments, metal chelates, protein and/or peptide ligands.

10. The substrate material according to at least one of claims 1 to 9, characterized in that the areas having affinity properties are constituted by metal chelates based on nickel/nitrilotriacetic acid chelates and the areas having anion-exchanging properties are constituted by materials capable of binding nucleic acids.

11. A process for evolutive optimization of biopolymers in which nucleic acids and their expression products are simultaneously bound to a substrate material and are selectively or concurrently isolated wherein a substrate material according to at least one of claims 1 to 10 is used.

12. The process according to claim 11, wherein the structural type representing the genotype is subjected to mutagenesis in a reaction chamber,
said structural type is subjected to at least one replication step, expressed,
and the expression product is bound to the substrate material along with the nucleic acids and examined.

## Revendications

1. Matériau support, qui présente simultanément au moins deux propriétés de fixation, pour assurer la fixation spécifique d'acides nucléiques et de leurs produits d'expression, où certaines zones de la surface du matériau support peuvent chacune fixer les acides nucléiques, tandis que les produits d'expression sont fixés par des zones localement séparées de la surface du matériau support, et une zone présente des propriétés d'affinité et une autre zone des propriétés d'échange d'ions ou d'affinité, ou encore une seule et même zone du matériau support fixe les acides nucléiques et les produits d'expression, et une partie de la zone présente des propriétés d'affinité, et une autre partie de cette zone présente des propriétés d'échange d'ions ou d'affinité.

2. Matériau support selon la revendication 1, caractérisé en ce que les différentes zones destinées à la fixation des acides nucléiques et de leurs produits d'expression se trouvent à une distance les unes des autres de 10 à 1000 µm et de préférence de 10 nm à 100 µm.

3. Matériau support selon l'une des revendications 1 et/ou 2, caractérisé en ce que la zone présentant des propriétés d'affinité permet des interactions réversibles avec les acides nucléiques fixés ou leurs produits d'expression, ce qui permet une élution d'au moins un type de molécule fixée.

4. Matériau support selon au moins l'une des revendications 1 à 3, caractérisé en ce que le matériau support est disposé d'une manière bidimensionnelle sur une membrane ou un composite membranaire, ou noyé dans une membrane, dans un capillaire ou sur une fibre ou dans un composite fibreux, tel qu'un non-tissé, ou leurs combinaisons.

5. Matériau support selon au moins l'une des revendications 1 à 4, caractérisé en ce que le matériau présentant des propriétés d'affinité et le matériau échangeur d'anions sont disposés dans une matrice telle qu'un non-tissé.

6. Matériau support selon au moins l'une des revendications 1 à 5, caractérisé en ce que la zone présentant des propriétés d'affinité est apte à la fixation spécifique d'au moins une protéine ou un peptide, et peut simultanément fixer des acides nucléiques.

7. Matériau- support selon au moins l'une des revendications 1 à 6, caractérisé en ce que les zones présentant des propriétés d'échange d'anions sont réalisées par des particules à gros pores ou à pores fermés, ayant subi une modification superficielle.

8. Matériau support selon la revendication 7, caractérisé en ce que la zone présentant des propriétés d'échange d'ions peut encore, pour une force ionique correspondant à du NaCl, 1 M, fixer des molécules d'ADN.

9. Matériau support selon au moins l'une des revendications 1 à 8, caractérisé en ce que la zone présentant des propriétés d'affinité est formée par des dérivés de la biotine, des dérivés de la streptavidine, des dérivés de l'avidine, des acides nucléiques, des fragments d'anticorps, des chélates métalliques, des protéines et/ou des ligands peptidiques.

10. Matériau support selon au moins l'une des revendications 1 à 9, caractérisé en ce que la zone présentant des propriétés d'affinité est formée de chélates métalliques à base de chélates nickel/acide nitrilotriacétique, et les zones présentant des propriétés d'échange d'anions sont formées par des matériaux convenant à la fixation d'acides nucléiques.

11. Procédé d'optimisation évolutive de biopolymères, dans lequel on fixe simultanément à un matériau support des acides nucléiques et leurs produits d'expression, et on les isole d'une manière sélective ou simultanément, en utilisant un matériau support selon au moins l'une des revendications 1 à 10.

12. Procédé selon la revendication 11, dans lequel, dans un espace réactionnel, le type de structure représentant le génotype est soumis à une mutagénèse, le type de structure est soumis à au moins une étape de réplication et exprimé, et le produit d'expression est fixé en même temps que les acides nucléiques au matériau support puis analysé.
